**Europäisches Patentamt**

**European Patent Office**

**Office européen des brevets**

(19)

(11) Veröffentlichungsnummer: **0 141 004**
**B1**

# EUROPÄISCHE PATENTSCHRIFT

(12)

(45) Veröffentlichungstag der Patentschrift:
07.01.88

(21) Anmeldenummer: 83810483.4

(22) Anmeldetag: 20.10.83

(51) Int. Cl.⁴: **A 61 F 2/00**

(54) **Knochenersatzmaterial auf der Basis natürlicher Knochen.**

(43) Veröffentlichungstag der Anmeldung:
15.05.85 Patentblatt 85/20

(45) Bekanntmachung des Hinweises auf die Patenterteilung:
07.01.88 Patentblatt 88/1

(84) Benannte Vertragsstaaten:
AT BE CH DE FR GB IT LI LU NL SE

(56) Entgegenhaltungen:
EP - A - 0 012 959
EP - A - 0 058 867
DE - A - 2 717 506
DE - A - 2 840 064
DE - A - 3 038 047
GB - A - 2 078 696
US - A - 4 314 380

(73) Patentinhaber: OSCOBAL AG, Bohnackerweg 1,
CH-2545 Selzach (CH)

(72) Erfinder: Mittelmeier, Heinz, Prof. Dr. med., Am
Gedünner 25, D-6650 Homburg-Schwarzenbach (DE)
Erfinder: Mittelmeier, Bernhard, D-6660 Zweibrücken
(DE)
Erfinder: Leu, Beat, Sonnenbergstrasse 68,
CH-6052 Hergiswil (CH)

(74) Vertreter: Seehof, Michel et al, c/o AMMANN
PATENTANWAELTE AG BERN Schwarztorstrasse 31,
CH-3001 Bern (CH)

**Beschreibung**

Die vorliegende Erfindung bezieht sich auf ein Verfahren zur Herstellung eines Knochenersatzmaterials auf der Basis natürlicher Knochen, wobei die Knochenstücke zum Entfernen der Weichteile einer Vorbehandlung unterzogen werden, anschliessend eine Enteiweissung mittels einer mindestens 10%igen $H_2O_2$-Lösung erfolgt und dann die Knochenstücke in einem Ofen einer Verbrennung unter Luftzufuhr und anschliessend einer Sinterung unterworfen werden.

Ein solches Verfahren ist aus der US-A-4 314 380 bekannt, wobei gemäss dieser Patentschrift ausschliesslich tierisches Knochenmaterial verwendet wird. Mit diesem Verfahren werden die organischen Substanzen, die Fremdkörperreaktionen auslösen können, vollständig entfernt, jedoch erreicht dieses Material die nötige mechanische Festigkeit nicht. Einerseits wurde vom Anmelder erkannt, dass der angegebene Temperaturverlauf im Verbrennungs- und Sinterofen nicht optimal ist und andererseits, dass die angegebene Backtemperatur zu niedrig ist, um eine genügende Verfestigung zu erzielen. Ausserdem benötigt die angegebene Vorbehandlung eine grosse Zeitdauer von bis zu drei Wochen und schlägt eine alkalische Lösung, $H_2O_2$–NaOH, vor, die zu einer Schwächung des inneren Mineralzusammenhangs führt und nach dem Brennen seine mechanische Festigkeit herabsetzt und überdies zu Negativwirkungen im Gewebe führen kann. Diese Patentschrift ist überdies auf die Herstellung eines Knochenersatzmaterials gerichtet, das in jedem Falle mit Atelocollagen durchtränkt wird, das durch die ungenügende Festigkeit des Zwischenproduktes bedingt ist.

Aus der DE-A-2 840 064 ist es bekannt, hydroxylapatithaltige Massen herzustellen, indem Zähne und/oder Zahnwurzeln und/oder tierische Knochen mittels einer 20%igen $H_2O_2$-Lösung enteiweisst und mittels Äther entfettet werden. Die Masse wird dann aufgeheizt und bei einer Temperatur von 800–1200 °C während 10 Min. bis 3 Stunden geglüht. Auch dieses Endprodukt besitzt keine ausreichende Festigkeit.

In der DE-C-961 654 ist ferner ein unter dem Namen «Kieler Knochenspan» bekanntes Knochenersatzmaterial beschrieben. Dieses heteroplastische Knochenersatzmaterial wird aus Kälbern und jungen Rindern gewonnen, bei dem durch besondere chemische Verfahren lösliche Proteine, Fette und Feuchtigkeit entfernt werden, so dass schliesslich lediglich das Gerüstwerk des Knochens verbleibt, welches aus der kollagenen Knochengrundsubstanz und den Knochenmineralien besteht. In der orthopädisch-chirurgischen Praxis konnte dieses Knochenersatzmaterial die darin gesetzten Erwartungen nicht erfüllen und umfangreiche histologische Untersuchungen haben ergeben, dass an der Oberfläche dieses Knochenersatzmaterials zwar teilweise eine Knochenablagerung stattfindet, dieselbe aber offensichtlich doch durch gewisse Fremdkörperreaktionen beeinträchtigt ist, welche auf das noch verbleibende Fremdkollagen zurückzuführen sind.

In den letzten beiden Jahrzehnten hat die Endoprothetik mit alloplastischem, körperverträglichem Fremdmaterial aus Metallen, Kunststoffen und mit Knochenzement stark zugenommen, da hiermit im Gelenkbereich relativ schnell gute Erfolge zu erzielen sind, wie sie durch Transplantationen von Knochengelenkteilen natürlicher Herkunft nicht erreicht werden können. Allerdings sind diese technischen Ersatzprodukte schliesslich doch mit Verschleissprozessen und vor allem erheblichen Verankerungsproblemen belastet und keiner natürlichen Regeneration zugänglich, so dass hier Grenzen gesetzt sind. Dies gilt vor allem im Bereich des Knochenschaftes, wo prothetische Überbrückungen sich nicht so gut bewährt haben und allenfalls als palliative Massnahmen bei Patienten mit malignen Knochentumoren und kurzer Lebenserwartung dienen.

Das ideale Ziel des Knochenersatzes ist immer, wenn möglich die Herbeiführung einer Regeneration mit natürlichen, vitalen, anpassungsfähigen Knochenstrukturen, insbesondere bei jüngeren Menschen mit langer Lebenserwartung. Dieses Ziel kann mit der Endoprothetik nicht erreicht werden, so wertvoll auch ihre Möglichkeiten sind.

In den letzten Jahren zeichnet sich die Tendenz ab, Knochenersatzmaterial aus synthetischen Kalziumphosphaten oder Hydroxylapatit herzustellen. Dieses Material hat den Vorteil, dass es Formstabilität besitzt, welche dazu dienen kann, der Knochenneubildung als Formmatrix zu dienen. Jedoch ist dieselbe nicht sehr belastungsfähig. Im Falle von Querschnittsknochendefekten muss deshalb dennoch eine abstützende Osteosynthese, gewöhnlich durch metallische Plattenüberbrückung der verbleibenden Stumpfenden zusätzlich vorgenommen werden, ähnlich wie dies auch für den eingangs beschriebenen «Kieler Span» zutrifft. Der Vorteil letzterem gegenüber besteht darin, dass die synthetische Apatitkeramik kein immunogenes Eiweisselement enthält. Andererseits besteht ein Nachteil darin, dass die synthetische Apatitkeramik die natürliche Knochenstruktur, insbesondere in der Kombination von Corticalis und Spongiosa nicht so gut nachahmen kann, wie dies beim «Kieler Knochenspan» als natürlichem Knochengewebe gegeben ist. Zur Erzielung gleicher Festigkeit ist eine grössere Dichte mit geringerer Porosierung erforderlich, welche der nachträglichen Erschliessung durch Einwachsen des Heilgewebes und der natürlichen Regeneratknochenbildung eher stärker im Wege steht als der «Kieler Knochenspan».

Zur Vermeidung der barriereartigen Hindernisse, welche die Verwendung von Knochenersatzmaterial auf natürlicher Basis oder spongiöser Apatitkeramik darstellt, wurde von der Anmelderin der Weg beschritten, eine Knocheninduktion durch ein Mischpräparat aus synthetischem Apatit und gereinigtem Kollagen in Vliesform zu erreichen, wobei das gereinigte, immunogenfreie Kollagen vorwiegend als Verteilungsträger wirkt (siehe EP-A-0 030 583). Dieses Knochenersatzmaterial zeigt zwar eine sehr gute Knochenbildung, welche rasch zum Aufbau eines natürlichen spongiösen Knochennetzes führt, und eignet sich gut zur Auffüllung von Knochenhöhlen und auch als Positionsplastik auf noch vorhandenen körpereigenen Knochen, beispielsweise bei Frakturen, Pseudarthrosen oder Versteifungsoperationen der Wirbelsäule, jedoch weniger gut zur Überbrückung freier Defektstrecken, wie sie beispielsweise bei Defektpseudarthrosen oder Querschnittsresektionen des Knochens bei Knochentumoren zustande kommen.

Damit besteht nach dem gegenwärtigen Stand der Technik das Bedürfnis nach einem formstabilen, als Matrix für die Knochenregeneration wirkenden Knochenersatzmaterial, welches jedoch, im Unterschied zum vorbekannten «Kieler Knochenspan» – absolut antigenfrei, also völlig frei von organischen Substanzen ist, möglichst den natürlichen Knochenstrukturen entspricht und beispielsweise natürlichen menschlichen Knochen identisch sein kann. Damit wäre es möglich, dem letzten Ziel nahe zu kommen, aufgrund von Erkrankungen zerstörte und operativ entfernte Knochenteile oder auch Gesamtknochen einem natürlichen Wiederaufbau zuzuführen. Obgleich mittels der vorgehend beschriebenen Apatitkeramik knochenähnliche Strukturen erreicht werden können, ist es damit jedoch nicht möglich, wirkliche natürliche Knochenstrukturen herzustellen, welche durch eine dichte stabile Knochenrinde (Corticalis) mit den entsprechenden feinen Gefässkanälchen (Volkmann'schen Kanälchen) und natürlichen Spongiosa-Strukturen ausgestattet sind und das Einwachsen von Heilgewebe und die Regeneration begünstigen und eine optimale Bioarchitektonik aufweisen.

Ausserdem sollte das Knochenersatzmaterial eine grössere Bruchfestigkeit als das eingangs erwähnte Material gemäss der US-A-4 314 380 aufweisen.

Ausgehend vom genannten Stand der Technik ist es daher Aufgabe der vorliegenden Erfindung ein Verfahren zur Herstellung von Knochenersatzmaterial anzugeben, das völlig antigenfrei ist und bessere mechanische und klinische Eigenschaften als das vorbekannte Material aufweist, um auch zur Überbrückung freier Defektstrecken zu dienen. Eine weitere Aufgabe liegt darin, das Herstellungsverfahren wesentlich zu beschleunigen.

Die erste Aufgabe wird mit den in Patentanspruch 1 genannten Merkmalen gelöst. Eine noch bessere Festigkeit wird durch die Merkmale der Patentansprüche 2 und 4 erzielt, während Patentanspruch 5 eine wesentliche Beschleunigung und damit Vereinfachung der Herstellung beschreibt. Weitere vorteilhafte Merkmale sind in den Patentansprüchen 3 und 6 bis 12 genannt.

Die Erfindung wird im Folgenden anhand von Ausführungsbeispielen näher erläutert.

Zusammenfassend kam man zu dem nachstehend ausführlich beschriebenen Verfahren sagen, dass Knochengewebe menschlichen, hauptsächlich jedoch tierischen Ursprungs, insbesondere von jungen Schweinen und Kälbern, zunächst einer schonenden Teilmazeration auf chemischem oder enzymatischem Wege und anschliessend einer schonenden Verbrennung der anorganischen Substanzen mit nachfolgender Glühsinterung der verbleibenden Mineralsubstanzen unterzogen wird.

Bei der gesamten Vorbehandlung und Mazeration ist darauf zu achten, dass eine Sprengwirkung auf die natürlichen Gerüststrukturen des Knochens sowohl durch Frieren als auch durch Kochen möglichst vermieden wird. Demzufolge wird das frische, im Obduktionssaal gewonnene menschliche oder auf dem Schlachthof gewonnene tierische Knochenmaterial im allgemeinen frisch verarbeitet, allenfalls nur kurzfristig bei Temperaturen knapp über dem Gefrierpunkt gelagert.

Beim ersten Verfahrensschritt erfolgt die weitere Bearbeitung dann jedoch am besten nach Erwärmen des Knochenmaterials auf etwa 40 bis 50 °C, um das im Knochengewebe sich befindliche Fettgewebe flüssig zu halten und damit leichter ausspülen zu können. Die Knochen werden hierzu deshalb am besten kurzfristig im Wasserbad auf diese Temperatur erwärmt.

Sodann erfolgt Befreiung von den anhaftenden Weichteilen (Muskeln, Knochenhaut und Gelenkknorpel) und Zersägung mit einer Knochenbandsäge in einer den Operationsbedürfnissen entsprechenden verschiedenartigen Grösse und Form. Danach erfolgt Entfernung des Markgewebes und -fettes grobmechanisch durch Abkratzen sowie anschliessend durch scharfes Absprühen mit Warmwasser-Duschstrahl, am besten auf einem Sieb. Anschliessend Wässern, so dass noch anhaftende Weichteile abschwemmen können. Anschliessend Dekantieren, eventuell nochmalige Sprühreinigung, Wässerung und danach schonende Trockenschleuderung bei Temperaturen von etwa 50 °C. Dadurch ergibt sich gegenüber dem Verfahren gemäss DE-C-961 654 eine Verkürzung von 2–3 Tagen auf 2–3 Stunden.

Im zweiten Schritt erfolgt die Feinmazeration in Wasserstoffsuperoxydlösung. Das Knochenmaterial wird dazu in Behältern mit 10–30%iger Wasserstoffsuperoxydlösung mit einer Schüttelmaschine schonend geschwenkt, was grosstechnisch auch durch Einbringen der Knochenstücke mit einem Korbsieb aus nichtrostendem Stahl oder Keramik erfolgen kann. Beim Eintauchen der Knochenstücke in die Wasserstoffsuperoxydlösung wird aus derselben Sauerstoffgas frei, welches zur Ablösung des Eiweisses und Fettgewebes von den Knochenoberflächen und den feinen Knochenkanälchen führt. Die abgelösten Partikel werden dabei durch das Sauerstoffgas an die Oberfläche geschwemmt. Zugleich erfährt das Knochengewebe dabei eine Entkeimung und Bleichung. Dieser Vorgang wird erforderlichenfalls noch ein- bis zweimal wiederholt, bis das Knochengewebe völlig sauber weiss erscheint. Anschliessend Dekantierung und Föntrocknung der Knochenstücke mit mässiger Temperatur (ca. 50 °C). Auch bei diesem Verfahrenschritt ergibt sich eine gleiche Zeitersparnis wie beim ersten Schritt.

Anschliessend erfolgt im 3. Schritt noch ein Feinentfettungsvorgang durch Eintauchen der getrockneten Knochenstücke in Äthyläther, in dem sich das Fettgewebe löst. Der Äther kann durch anschliessende Redestillation zurückgewonnen werden. Nach Dekantieren des Äthers bzw. Herausnehmen des Korbsiebes mit den Knochenstücken erfolgt zunächst Lufttrocknung und zwecks Vermeidung einer Explosionsgefahr erst nach weitgehendem Abdunsten des Äthers Föntrocknung mit ca. 50 °C. Nach obigem Verfahren können die ersten drei Verfahrensschritte nach ca. 6 Stunden vollendet sein, gegenüber 6–8 Tagen beim Verfahren gemäss DE-C-961 654.

Sodann erfolgt als 4. Schritt der Verbrennungsprozess der restlichen Kollagensubstanzen des Knochengerüstes in einem Keramikofen, der eine genaue Temperaturregelungsmöglichkeit aufweist. Die anschliessende Erhitzung erfolgt unter Luftzutritt, so dass eine regelrechte Verbrennung der organischen Substanzen und keine Verkokung eintritt.

Es ist nun wesentlich, dass die Verbrennung mit langsam ansteigender Temperatur erfolgt, so dass die Kollagensubstanz zunächst noch weiter austrocknen und keinesfalls eine Wasserverdampfung im Inneren zur Sprengung der Kristallstrukturen führen kann, desgleichen, dass die Verbrennungsgase keinen starken Dampfdruck erzeugen und schonend verflüchtigen. Die Temperatur wird deshalb so gesteuert, dass sie langsam ansteigt, so dass erst etwa nach einer Viertelstunde 400 °C, nach einer halben Stunde 800 °C und schliesslich nach einer Stunde die Temperatur zwischen 1000° und 1500 °C, vorzugsweise im Bereich von 1250 °C, erreicht werden.

Dabei ist wesentlich zu erkennen, dass die Erhitzung nicht nur zum Zwecke der vollständigen Enteiweissung durchgeführt wird, sondern anschliessend eine langdauernde Erhitzung zum Zwecke der keramischen Sinterung der natürlichen Mineralstrukturen des Knochengewebes durchgeführt wird. Damit wird erreicht, dass das durch einen kurzzeitigen Verbrennungsprozess aufgrund des Verlustes der inneren Kollagen-Faserverbindung sehr brüchige Material sich durch Sinterung wieder verfestigt.

Mechanische Untersuchungen haben ergeben, dass ein gleichartig vorbehandeltes Spongiosagewebe nach einer relativ kurzzeitigen Glühung bis zu 2 Stunden, wie früher durchgeführt, nur eine geringe Festigkeit besitzt und leicht zerdrückt werden kann. Es ist damit zum Formaufbau von Knochendefekten nicht gut geeignet. Dagegen wird durch ein an den Verbrennungsprozess angeschlossenes mehrstündiges keramisches Sintern dem Knochengerüst wieder eine erhöhte Festigkeit verliehen. Diese reicht natürlich nicht an die hohe Festigkeit des frischen natürlichen Knochengewebes heran, welches durch den Gehalt von Kollagenfasern einem sehr festen Verbundwerkstoff entspricht. Durch die Sinterung kann jedoch eine für praktische Zwecke ausreichende Festigkeit wieder gewonnen werden, welche den Festigkeitsverlust durch Vernichtung der Kollagenfasern in ausreichender Weise wieder ausgleicht.

Nach durchgeführten Festigkeitsmessungen sollte eine mindestens zweistündige bis zwanzigstündige Sinterung bei etwa 1250 °C erfolgen, vorzugsweise eine vier-acht-zehnstündige Sinterung.

Es sollte aber auch keine plötzliche Herausnahme und Abkühlung der Knochenstücke aus dem Keramikofen erfolgen, weil es dadurch zu «Abschreckungsbrüchen» der gesinterten Knochenstruktur mit Festigkeitsverlust kommen kann. Vielmehr ist es günstiger, den Knochen langsam über mehrere Stunden hinweg durch Zurückfahren der Temperatur im Keramikofen abkühlen zu lassen. Der Abkühlungsprozess soll vorzugsweise bis zu 24 Stunden dauern.

Durch den Keramisierungsprozess wird das Knochengewebe natürlich auch keimfrei gemacht, also sterilisiert. Es kann demzufolge mit einem sterilen Instrument in einen sterilen Behälter gebracht und zur Verwendung verschickt werden.

Es ist aber auch möglich, das abgekühlte Knochenmaterial in strahlendurchgängigen Plastikbehältern zu verpacken und zusätzlich einer Strahlensterilisation zu unterziehen.

Das Knochenmaterial unterliegt im Zuge des Sinterungsverfahrens einer Schrumpfung, welche sich nicht nur bezüglich der Abmessungen der Knochenstücke insgesamt zeigt, sondern vor allem dazu führt, dass die Wandgefächer des spongiösen Knochenmaterials, welche nach SCHWEIBERGER dem Einwachsen des Heilgewebes und der anschliessenden Remodellierung des Knochengewebes einen barriereartigen Widerstand entgegenstellen, sich zu mehr strangförmigen Knochenbälkchen zusammenzuziehen. Das derart hergestellte Knochengerüstwerk zeigt damit eine relative Erweiterung der Maschenräume bei relativer Verschmächtigung der Bälkchen, was für die Erschliessung, nämlich das Einwachsen des Heilgewebes und die Remodellierung neugebildeten lebenden Knochens in denselben günstig ist. Die Volumenschwindung der verbleibenden Mineralsubstanz geht jedoch aufgrund der Verdichtung nicht mit einer Schwächung, sondern umgekehrt mit einer mechanischen Verstärkung einher. Es wird also in doppelter Hinsicht ein günstiger Effekt erzielt.

Weitere Untersuchungen des derart gewonnenen Knochenmaterials mit dem Rasterelektronenmikroskop zeigen, dass die Kristallanordnung des Knochengewebes durch den schonenden Verfahrensprozess erhalten bleibt.

Auch chemisch bleibt das im natürlichen Knochenmineral überwiegende Hydroxylapatit weitgehend erhalten. Dies zeigt sich in röntgenkristallographischen Untersuchungen, welche eine unverändert ausgeprägte Apatitstruktur zeigen.

Nach Einbringen des derart gewonnenen Knochens in destilliertes Wasser zeigt sich eine leicht alkalische Reaktion, welche annehmen lässt, dass bei dem Sinterungsprozess in geringem Umfang auch Calciumoxyd freigesetzt wird, welches nach Einbringen in wässrige Lösungen als Calciumhydroxyd die leicht alkalische pH-Änderung bewirkt. Auch diese erscheint günstig, da erfahrungsgemäss die Knochenneubildung ein leicht alkalisches Milieu benötigt. Möglicherweise entstehen auch in geringem Umfang leichter lösliche niedrige Calciumphosphate, welche erfahrungsgemäss auf chemische Weise das Heilgewebe zur Knochenbildung anregen.

Mikroradiographische und histologische Untersuchungen zeigen, dass es nach Einpflanzen des derart gewonnenen Knochenmaterials in Defekthöhlen in dem einwachsenden Heilgewebe (Granulationsgewebe) keine Fremdkörperreaktionen gibt und das Heilgewebe rasch zur Knochenneubildung angeregt wird. Diese erfolgt sowohl durch Abscheidung lamellösen Knochens durch knochenbildende Zellen (Osteoblasten) unmittelbar auf den Bälkchen des implantierten Knochenmaterials als auch auf dem Markraum-Maschenwerk. Aufgrund der von Fremdkörperreaktionen ungestörten Organisationsprozesse kommt es auch viel rascher zur Wiederherstellung natürlicher Knochen- und Markstrukturen, so dass teilweise schon nach wenigen Wochen die Markräume wieder mit blutbildendem Knochenmark gefüllt sind.

Das derart gewonnene Knochenmaterial eignet sich nicht nur zur Auffüllung von Knochendefekthöhlen oder zur Oberflächenanlagerung, sondern insbesondere auch zur Überbrückung grösserer Knochendefekte, bei denen Formstabilität und auch eine gewisse leichte mechanische Belastbarkeit gefordert werden. Es dient als Matrix für den durch das Implantatmaterial selbst angeregten natürlichen Knochenwiederaufbau. Dadurch, dass es volumenmässig zu dünneren Bälkchen zusammengesintert ist, kann sich in den Maschenräumen und an der Oberfläche das neugebildete Knochengewebe besser entfalten und rascher die funktionelle Belastung wieder übernehmen als dies bei nur teilmazerierten Knochenspänen mit relativ sperrigen Knochenfächern der Fall ist.

Das Material könnte in gemahlener Form auch zur Bioaktivierung von Knochenzement, plastischen Verankerungsteilen von Gelenkendoprothesen oder anderen Knochenersatzmaterialien Verwendung finden.

Der knochenbildende Effekt kann durch Beimpfen mit autologem Knochenmarkextrakt noch gesteigert werden. Es besteht auch die Möglichkeit einer präoperativen Beschickung mit Fibrin oder gelöstem Kollagen sowie verschiedenartigen Antibiotika, in Pulverform oder durch Eintauchen in Antibiotikalösung.

Wesentlich erscheint, dass beim Gesamtersatz menschlicher Knochen, bei dem auf menschliche Ausgangsknochen (von Amputationspräparaten oder Leichen) zurückgegriffen werden müsste, die Möglichkeit besteht, die Gelenkflächen mit Gelenkprothesen zu versehen, beispielsweise am Hüftkopf, durch Aufsetzen (Zementieren) eines Gelenkflächenersatzes bzw. Hüftteil- oder Totalprothese.

Um das bessere Einwachsen von Gesamtknochen zu ermöglichen, sollten dieselben am besten bereits vor der Fertigungsprozedur mit zahlreichen Bohrlöchern von etwa 3 mm Durchmesser und mit einem Rasterabstand von etwa 1 cm versehen werden, um das Einwachsen des Heilgewebes (Granulationsgewebes) zu erleichtern.

Auch besteht die Möglichkeit, zur Erhöhung der Stabilität den Knochen vorher mit einer Markraumschiene (Marknagel) zu versehen oder aber an der Oberfläche eine Stabilisierungsschiene (Osteosyntheseplatte) anzuschrauben.

Das Implantat kann erst nach ausreichender natürlicher Knochenregeneration einer zunehmenden Belastung unterzogen werden.

Nebst der Form von bestimmten Knochen oder Knochenstücken kann das Knochenersatzmaterial auch als Späne, Schnitzel, Würfel oder dergleichen vorliegen.

## Patentansprüche

1. Verfahren zur Herstellung eines Knochenersatzmaterials auf der Basis natürlicher Knochen, wobei die Knochenstücke zum Entfernen der Weichteile einer Vorbehandlung unterzogen werden, anschliessend eine Enteiweissung mittels einer mindestens 10%igen $H_2O_2$-Lösung erfolgt und dann die Knochenstücke in einem Ofen einer Verbrennung unter Luftzufuhr und anschliessend einer Sinterung unterworfen werden, dadurch gekennzeichnet, dass nach der Enteiweissung und vor der Verbrennung eine Entfettung mittels Äther erfolgt und dass, gerechnet vom Zeitpunkt des Einschubes der vorbehandelten Knochenstücke an, die Temperatur im Ofen derart geregelt wird, dass nach ca. 15 Min. 400 °C, nach ca. 30 Min. 800 °C und nach einer Stunde eine Temperatur zwischen 1000 °C und 1500 °C erreicht wird und die Sinterung während mindestens 2 Stunden durchgeführt wird.

2. Verfahren nach Anspruch 1, dadurch gekennzeichnet, dass die Ofentemperatur nach einer Stunde 1200–1300 °C, vorzugsweise 1250 °C, beträgt und die Sinterung während 4–18 Stunden durchgeführt wird.

3. Verfahren nach Anspruch 1 oder 2, dadurch gekennzeichnet, dass als Ausgangsmaterial menschliche Knochen verwendet werden.

4. Verfahren nach einem der Ansprüche 1 bis 3, dadurch gekennzeichnet, dass die Abkühlungsphase nach der Sinterung bis zu 24 Stunden beträgt.

5. Verfahren nach einem der Ansprüche 1 bis 4, dadurch gekennzeichnet, dass die Vorbehandlung aus einem Erwärmen der Knochenstücke in einem Wasserbad auf eine Temperatur von 40 °C bis 50 °C, einer mechanischen Entfernung der Weichteile und Absprühen mit einem scharfen Wasserstrahl und einem Trockenschleudern bei ca. 50 °C besteht und die Enteiweissung mittels einer 10 bis 30%igen $H_2O_2$-Lösung in einer Schüttelmaschine und anschliessender Trocknung in einem Warmluftstrom durchgeführt wird.

6. Verfahren nach einem der Ansprüche 1 bis 5, dadurch gekennzeichnet, dass das Knochenersatzmaterial einer Strahlensterilisation unterzogen wird.

7. Verfahren nach einem der Ansprüche 1 bis 6, dadurch gekennzeichnet, dass das Knochenersatzmaterial mit Fibrin oder gelöstem Kollagen und/oder mit Antibiotika in Pulver- oder gelöster Form beschickt und/oder mit autologem Knochenmarkextrakt beimpft wird.

8. Verfahren nach einem der Ansprüche 1 bis 7, dadurch gekennzeichnet, dass das in Form eines Knochenersatzteils vorliegende Material vor dem Verbrennen und Sintern mit Löchern, vorzugsweise mit einem Durchmesser von 3 mm und einem Rasterabstand von 10 mm, versehen ist.

9. Verfahren nach einem der Ansprüche 1 bis 7, dadurch gekennzeichnet, dass das Material wie das zu ersetzende Knochenteil ausgebildet wird.

10. Verfahren nach einem der Ansprüche 1 bis 7, dadurch gekennzeichnet, dass das Material mit einem künstlichen Gelenk versehen wird.

11. Verfahren nach einem der Ansprüche 1 bis 7, dadurch gekennzeichnet, dass das Material mit einer Markraumschiene oder einem -nagel oder mit einer Osteosyntheseplatte versehen wird.

12. Verfahren nach einem der Ansprüche 1 bis 7, dadurch gekennzeichnet, dass das Material in gemahlener Form, in Form von Spänen, Schnitzeln oder Würfeln hergestellt wird.

## Revendications

1. Procédé de fabrication d'un matériau de remplacement des os à base d'os naturels, dans lequel les parties d'os sont soumises à un prétraitement pour enlever les parties tendres puis à une élimination de l'albumine à l'aide d'une solution d'au moins 10% de $H_2O_2$, les parties d'os étant ensuite soumises dans un four à une combustion avec apport d'air puis à un frittage, caractérisé en ce qu'après l'élimination de l'albumine et avant la combustion, un dégraissage à l'aide d'éther est effectué et en ce que, calculé à partir du moment de l'introduction des parties d'os prétraitées, la température dans le four est réglée de manière à atteindre 400 °C après 15 min, 800 °C après 30 min et une température comprise entre 1000 °C et 1500 °C après une heure, le frittage étant exécuté pendant au moins deux heures.

2. Procédé selon la revendication 1, caractérisé en ce que la température du four est de 1200–1300 °C, de préférence 1250 °C, après une heure et le frittage est exécuté pendant 4–18 heures.

3. Procédé selon la revendication 1 ou 2, caractérisé en ce que des os humains sont utilisés comme matériau de départ.

4. Procédé selon l'une des revendications 1 à 3, caractérisé en ce que la phase de refroidissement après le frittage peut être de 24 heures.

5. Procédé selon l'une des revendications 1 à 4, caractérisé en ce que le prétraitement consiste en un réchauffement des parties d'os dans un bain d'eau à une température de 40 °C à 50 °C, en un enlèvement mécanique des parties tendres, en un giclage avec un intense jet d'eau et en un séchage par essorage centrifuge à environ 50 °C et en ce que l'élimination de l'albumine est exécutée à l'aide d'une solution de 10 à 30% de $H_2O_2$ dans une machine à secouer puis d'un séchage dans un courant d'air chaud.

6. Procédé selon l'une des revendications 1 à 5, caractérisé en ce que le matériau de remplacement des os est soumis à une stérilisation par radiation.

7. Procédé selon l'une des revendications 1 à 6, caractérisé en ce que le matériau de remplacement des os est chargé avec de la fibrine ou collagène dissous et/ou avec un antibiotique dissous ou sous forme de poudre et/ou inoculé avec un extrait de moelle épinière autologue.

8. Procédé selon l'une des revendications 1 à 7, caractérisé en ce que le matériau présent sous forme de matériau de remplacement des os est pourvu avant la combustion et le frittage de trous ayant de préférence un diamètre de 3 mm et une distance de trame de 10 mm.

9. Procédé selon l'une des revendications 1 à 7, caractérisé en ce que, le matériau est façonné pour avoir la forme de la partie osseuse à remplacer.

10. Procédé selon l'une des revendications 1 à 7, caractérisé en ce que le matériau est pourvu d'une articulation artificielle.

11. Procédé selon l'une des revendications 1 à 7, caractérisé en ce que le matériau est pourvu d'un rail d'espace médullaire ou d'un clou intra-médullaire ou d'une plaque d'ostéosynthèse.

12. Procédé selon l'une des revendications 1 à 7, caractérisé en ce que le matériau est fabriqué sous forme moulue, de copeaux, de rondelles ou de cubes.

## Claims

1. Process of production of a bone substitute material based on natural bones, in which the bone pieces are submitted to a pretreatment for removing the soft parts, then to dealbumination by means of a solution of at least 10% $H_2O_2$, the bone pieces being then submitted in a stove to a combustion under air supply and then to sintering, characterized in that after dealbumination and before combustion, a degreasing by means of ether takes place and in that, calculated from the moment of the introduction of the pretreated bone pieces, the temperature in the stove is adjusted to be at 400 °C after 15 min, 800 °C after 30 min and to be comprised between 1000–1500 °C, the sintering being executed during at least two hours.

2. Process according to claim 1, characterized in that the temperature in the stove is at 1200–1300 °C, preferably at 1250 °C after one hour and the sintering is executed during 4–18 hours.

3. Process according to claim 1 or 2, characterized in that human bones are utilized as initial material.

4. Process according to one of the claims 1 to 3, characterized in that the cooling phase after the sintering lasts up to 24 hours.

5. Process according to one of the claims 1 to 4, characterized in that the pretreatment consists in a heating of the bone pieces in a water bath at a temperature of 40 °C to 50 °C, a mechanical removing of the soft parts and spraying with a strong water stream and a spin drying at 50 °C and in that the dealbumination is executed by means of a solution of 10 to 30% $H_2O_2$ in a shaking machine and by subsequent drying in a warm air stream.

6. Process according to one of the claims 1 to 5, characterized in that the bone substitute material is submitted to a sterilization by radiations.

7. Process according to one of the claims 1 to 6, characterized in that the bone substitute material is loaded with fibrin or dissolved collagen and/or with antibiotics in powder or dissolved form and/or inoculated with autologous bone marrow extract.

8. Process according to one of the claims 1 to 7, characterised in that the material present in form of bone substitute material is provided before combustion and sintering with holes having preferably a diameter of 3 mm and a screen distance of 10 mm.

9. Process according to one of the claims 1 to 7, characterized in that the material is shaped like the bone part to be replaced.

10. Process according to one of the claims 1 to 7, characterized in that the material is provided with an artificial articulation.

11. Process according to one of the claims 1 to 7, characterized in that the material is provided with a marrow space rail or a intramedullary nail or with an osteosynthesis plate.

12. Process according to one of the claims 1 to 7, characterized in that the material is produced in grinded form, in form of chip, slices or cubes.